Europäisches Patentamt

European Patent Office

Office européen des brevets

(19)

(11) Veröffentlichungsnummer: **0 517 204 A1**

(12)

# EUROPÄISCHE PATENTANMELDUNG

(21) Anmeldenummer: **92109432.2**

(51) Int. Cl.5: **A61F 13/58**

(22) Anmeldetag: **04.06.92**

(30) Priorität: **07.06.91 DE 4118859**

(43) Veröffentlichungstag der Anmeldung:
**09.12.92 Patentblatt 92/50**

(84) Benannte Vertragsstaaten:
**AT CH DE ES FR GB IT LI NL PT**

(71) Anmelder: **VP - SCHICKEDANZ AG**
**Schoppershofstrasse 80**
**W-8500 Nürnberg 1(DE)**

(72) Erfinder: **Petranyi, Pal, Dr.**
**Schweriner Strasse 17**
**W-8500 Nürnberg(DE)**
Erfinder: **Rink, Norbert, Dr.**
**Wodanstrasse 17**
**W-8508 Wendelstein 2(DE)**

(74) Vertreter: **Rau, Manfred, Dr. Dipl.-Ing. et al**
**Patentanwälte Rau, Schneck & Hübner,**
**Königstrasse 2**
**W-8500 Nürnberg 1(DE)**

(54) **Mehrmals verschliessbare Windel.**

(57) Es wird eine mehrmals verschließbare Windel beschrieben mit
- einer flüssigkeitsdurchlässigen oberen Abdeckung,
- einer flüssigkeitsdichten unten angeordneten Wäscheschutzfolie (WS-Folie (1)),
- einer dazwischen angeordneten Saugschicht (2),

sowie
- wenigstens zwei Klebebändern (7,8) an einem Ende der Windel, deren Freienden nach dem Anlegen der Windel an der äußeren Oberfläche der WS-Folie (1) am gegenüberliegenden Ende der Windel ein- oder mehrmals festgeklebt werden. Dabei ist die WS-Folie (1) dort, wo die Freienden der Klebebänder (11,12) zum Verschließen der Windel festgeklebt werden, an der Folien-Innenseite durch eine aufkaschierte Schicht verstärkt. Diese aufkaschierte Verstärkungsschicht (14) besteht aus Papier oder Vliesstoff.

EP 0 517 204 A1

Die Erfindung betrifft eine mehrmals verschließbare Windel mit
- einer flüssigkeitsdurchlässigen oberen Abdeckung,
- einer flüssigkeitsdichten unten angeordneten Wäscheschutzfolie (WS-Folie),
- einer dazwischen angeordneten Saugschicht,

sowie
- wenigstens zwei Klebebändern an einem Ende der Windel, deren Freienden nach dem Anlegen der Windel an der äußeren Oberfläche der WS-Folie am gegenüberliegenden Ende der Windel ein- oder mehrmals festgeklebt werden.

Unter dem Ausdruck "Windel" werden hierbei alle sogenannten Wegwerfwindeln verstanden, also sowohl Kinderwindeln wie auch Erwachsenenwindeln. Die Windeln bestehen aus den beschriebenen mehrschichtigen Gebilden und sie sind in der Regel so geschnitten, daß sie beim Anlegen die Form eines Höschens annehmen. Das Höschen wird nach dem Anlegen mit den erwähnten Klebebändern verschlossen, wobei die Forderung gestellt werden muß, daß die mit ihren Freienden auf die gegenüberliegende Folie aufgeklebten Bänder auch wieder abgelöst und danach auf dieselbe oder eine nur wenig verschiedene Stelle wieder aufgeklebt werden können. Es ist dies erforderlich, um der Windel beim Anlegen die richtige Paßform zu erteilen oder auch um überprüfen zu können, ob die Windel noch sauber ist oder gewechselt werden muß.

Das mehrmalige Aufkleben und Wiederablösen der Verschlußbänder belastet die dünne Wäscheschutzfolie außerordentlich. Die Belastung ist dabei häufig so stark, daß die Folie einreißt, worauf die Windel nicht weiter verwendbar ist. Es sind deshalb schon mehrere Vorschläge gemacht worden, die Wäscheschutzfolie zumindest in dem Bereich, in dem sie durch die aufzuklebenden Verschlußbänder besonders belastet wird, zu verstärken. Die meisten Vorschläge dieser Art gehen dahin, auf die äußere Oberfläche der Wäscheschutzfolie ein besonderes Band oder Etikett aufzukleben oder sonstwie aufzukaschieren, dessen Reißfestigkeit und Schälfestigkeit größer ist als die der Wäscheschutzfolie. Ein solches Band verteuert und versteift jedoch die Windel an der Vorderseite, so daß manche Windelhersteller diese Lösung nicht anwenden möchten.

In der DE-OS 30 22 916 ist eine wiederholt verschließbare Windel beschrieben, bei der sich an der Innenseite der Wäscheschutzfolie eine stoffschlüssige daran befestigte Schicht aus einem Heißschmelzkleber befindet. Diese Schicht soll geeignet sein, die Folie an der betreffenden Stelle derart zu verstärken, daß sie der Belastung bei mehrmaligem Ankleben und Wiederablösen der Klebebänder standhält. Praktische Versuche und Erfahrungen mit dieser Lösung haben jedoch gezeigt, daß der beschriebene Weg nur dann hinreichend erfolgreich ist, wenn die aufgebrachte Heißschmelzkleberschicht verhältnismäßig dick ist. In der Schrift werden Schichtstärken bis 127 $\mu$m angegeben, tatsächlich sind jedoch je nach angewandter WS-Folie sogar noch höhere Schichtstärken erforderlich.

In der EP-PS 02 87 767 ist eine Weiterentwicklung von Windeln mit derartigen WS-Folien beschrieben, die darin besteht, daß die Verstärkung in Form einer glatten Schicht, vorzugsweise einer aufkaschierten zusätzlichen Folienschicht an der Innenseite der Kunststoff-Folie des Vorderteils angebracht ist. Als Folienschicht wird insbesondere eine Polypropylenfolie empfohlen, die mit Hilfe eines druckempfindlichen Klebers an der entsprechenden Stelle der WS-Folie angebracht ist. Die so erreichte lösung des Problems ist durchaus zufriedenstellend, jedoch muß bedacht werden, daß der Kostenaufwand für die zusätzliche Folie beachtlich ist.

Der Erfindung liegt die Aufgabe zugrunde, eine adäquate lösung des geschilderten Problems zu finden, wobei jedoch die Kosten der zusätzlich erforderlichen Maßnahmen noch weiter gesenkt werden. Es muß in diesem Zusammenhang bedacht werden, daß Windeln der hier angesprochenen Art zur Gruppe der sogenannten Wegwerfprodukte gehören, welche ihrer Bestimmung entsprechend nur einmal verwendet werden. Produkte dieser Art sind wirtschaftlich nur dann zu vertreten, wenn ihr Preis so niedrig wie irgendmöglich ist.

Zur Lösung der gestellten Aufgabe wird von einer mehrmals verschließbaren Windel mit folgenden bekannten Merkmalen ausgegangen:
- einer flüssigkeitsdurchlässigen oberen Abdeckung,
- einer flüssigkeitsdichten unten angeordneten Wäscheschutzfolie (WS-Folie),
- einer dazwischen angeordneten Saugschicht,
sowie
- wenigstens zwei Klebebändern an einem Ende der Windel, deren Freienden nach dem Anlegen der Windel an der äußeren Oberfläche der WS-Folie am gegenüberliegenden Ende der Windel ein- oder mehrmals festgeklebt werden,
wobei
- die WS-Folie dort, wo die Freienden der Klebebänder zum Verschließen der Windel festgeklebt werden, an der Folieninnenseite durch eine aufkaschierte Schicht verstärkt ist.

Zur lösung dieser Aufgabe wird vorgeschlagen, daß die auf der Innenseite der WS-Folie angeordnete Verstärkungsschicht aus Papier oder Vliesstoff besteht. Bei Versuchen wurde überraschenderweise festgestellt, daß auch diese äußerst preiswerten und zumindest wenn sie auf Zellulose-Basis aufgebaut sind, biologisch abbaubaren Werkstoffe in der Lage sind, die dünne WS-Folie derart zu verstärken, daß sie einem mehrmaligen Ankleben und Wiederablösen der Klebebänder ohne Zerstörung standhält. Bei näheren Untersuchungen hat sich herausgestellt, daß die Verstärkungsschicht vorteilhafterweise aus Papier mit einen Flächengewicht von 15 - 80 g/m$^2$ besteht. Innerhalb dieser Grenzen haben sich folgende Papierqualitäten als geeignet erwiesen:

Seidenpapier mit 18 - 25 g/m$^2$

Kraftpapier mit 30 - 50 g/m$^2$

Pergaminpapier mit 25 - 40 g/m$^2$ oder

satiniertes Papier mit 30 - 50 g/m$^2$

Anstelle von Papier können auch Vliesstoffe verwendet werden, wobei sich folgende Qualitäten ausgezeichnet haben:

ein Zellwollvliesstoff mit 14 - 30 g/m$^2$

ein Polyestervliesstoff mit 10 - 30 g/m$^2$

ein Polyolefinvliesstoff mit 14 - 130 g/m$^2$·

Da Vliesstoffe dieser Art in der Regel weicher sind als die zuvor genannten Papiersorten, erscheinen derart verstärkte Windeln als äußerst komfortabel. Die genannten Stoffe, also sowohl die Papiere als auch die Vliesstoffsorten, werden mit geeigneten herkömmlichen und käuflichen Klebstoffen, beispielsweise Sprühklebstoffen oder vorzugsweise Heißschmelzklebern an der Innenseite der WS-Folie befestigt. Die Klebstoffmenge kann hierbei auf ein äußerstes Minimum reduziert werden; sie muß gerade ausreichen, um die gewünschte Haftung zu erzielen. Anstelle der erwähnten Klebstoffe ist es bei Verwendung von Vliesstoffen aber auch möglich, diese an der Folie festzuschweißen, etwa durch Ultraschallbehandlung. Alle technisch möglichen und bekannten Befestigungsarten werden vorliegend unter dem Oberbegriff "Aufkaschieren" verstanden.

Vergleichsversuche haben ergeben, daß die erreichte Folienverstärkung hinsichtlich der Abschälfestigkeit von Verschlußetiketten bei Verwendung von Papier oder Vliesstoff zumindest in der gleichen Größenordnung liegt, wie sie auch mit Heißschmelzaufkleberauflagen oder zusätzlichen Folien erreicht wird. In aller Regel ist die Festigkeitssteigerung bei Verwendung von Papier aber sogar noch größer. Um dies zu demonstrieren, wurde eine Methode gewählt, bei der die zu untersuchende WS-Folie zunächst durch Hinterkaschieren mit Papier, Vliesstoff, Kunststoff-Folie oder Schmelzkleber verstärkt wird. Danach wird eine Mehrzahl von derart vorbehandelten Folienabschnitten, etwa 10 Stück, mit einem handelsüblichen Verschlußband für Windeln beklebt. Dabei wird ein vorbestimmter Anpreßdruck angewandt, indem das Band mit einer Gummirolle bestimmter Geschwindigkeit angerollt wird. Nachfolgend wird das Etikett entweder sofort oder nach einer Wärmebehandlung von 16 Stunden bei 40° von Hand abgezogen. Der Abzugswinkel liegt dabei stets bei ca. 140 bis 160°; die Abzugsgeschwindigkeit beträgt ca. 100 cm/sek. Als Testergebnis wird vermerkt, wieviel Proben aus einer Gruppe von jeweils 10 Stück die Prüfung ohne Beschädigung überstehen, wenn jeweils einmal, dreimal, fünfmal oder zehnmal abgezogen wird. Bei Verwendung einer 25 μ-Polyethylenfolie hat sich dabei das aus der nachfolgenden Tabelle wiedergegebene Bild ergeben.

| | 1x | 3x | 5x | 10x Abz. |
|---|---|---|---|---|
| 25μ PE-Folie ohne Verstärkung | - | - | - | - |
| 25μ PE-Folie, mit PP-Folie hinterklebt | 10 | 9 | 8 | 8 |
| 25μ PE-Folie mit Seidenpapier 20 g/m$^2$ hinterklebt | 10 | 9 | 8 | 9 |
| 25μ PE-Folie mit Kraftpapier 40 g/m$^2$ hinterklebt | 10 | 10 | 9 | 9 |
| 25μ PE-Folie mit satiniertem Papier 40 g/m$^2$ hinterklebt | 10 | 10 | 8 | 9 |
| 25μ PE-Folie mit Pergamin 40 g/m$^2$ | 10 | 10 | 8 | 9 |
| 25μ PE-Folie mit Polyestervliesstoff 25 g/m$^2$ | 10 | 10 | 7 | 8 |

Die in der Tabelle angegebenen Zahlen zeigen die Anzahl von Proben aus jeweils 10 Stück, welche die Prüfung ohne Beschädigung überstanden haben. Es ist ersichtlich, daß die mit Papier oder Polyestervliesstoff hinterkaschierten Proben diesen äußerst scharfen Test mindestens ebensogut, teilweise sogar besser überstanden haben als solche Proben, die mit einer PP-Folie hinterklebt waren.

Die beigefügte Windel zeigt eine Kinderwindel in Draufsicht auf die Innenseite.

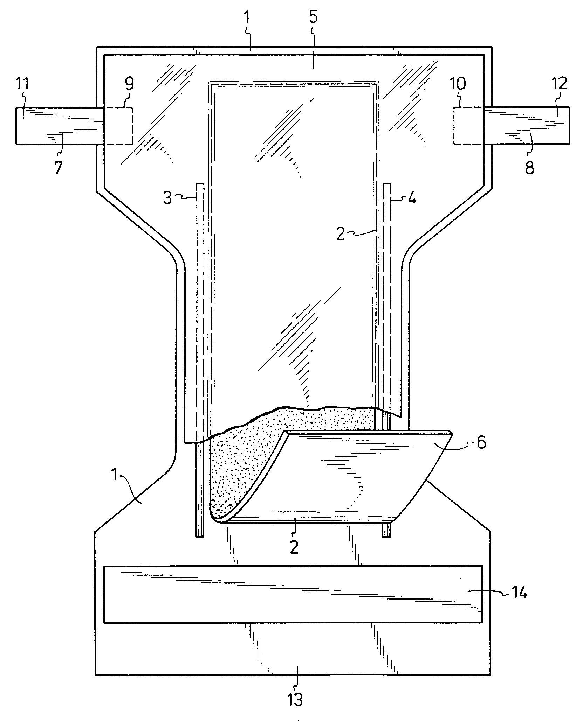

Die Windel besteht aus einem Doppel-T-förmigen Zuschnitt 1 aus einer WS-Folie. Als WS-Folie wird in

EP 0 517 204 A1

der Regel eine Polyethylenfolie von 20-30 $\mu$m Dicke verwendet. Auf der Folie ist ein Saugkissen 2 angeordnet, welches in bekannter Weise aus Zellstoff-Flocken, ggf. in Mischung mit Quellstoffteilchen besteht. Rechts und links vom Saugkissen 2 befinden sich elastische Gummibänder 3 und 4, welche im gestreckten Zustand auf die WS-Folie aufgeleimt sind. Die Gummibänder haben den Zweck, die Windel im Schrittbereich zu rüschen, wenn sie zum Gebrauch dem Träger angelegt wird. Das Paket aus Wäscheschutzfolie und Saugkissen ist alsdann mit einer oberen Schicht 5 aus Vliesstoff abgedeckt, welche Schicht mit dem Zuschnitt 1 ringsum verleimt ist. In der Zeichnung ist die Vliesstoffschicht 5 unten angerissen und der untere Abschnitt 6 des Saugkissens 2 ist hochgeklappt.

Am oberen Ende der Windel sind zwei Verschlußstreifen 7 und 8 angeordnet, die mit ihren Enden 9 und 10 fest am Zuschnitt 1 verklebt sind. Wenn die Windel dem Träger angelegt ist, werden die Freienden 11 und 12 an der äußeren Oberfläche des Windelzuschnitts befestigt und zwar am entgegengesetzten Teil der Windel, welches in der Zeichnung mit 13 bezeichnet ist. An diesem Teil muß die WS-Folie verstärkt werden, um widerstandsfähig gegen das Festkleben und Wiederlösen der Etiketten 7 und 8 zu sein. Hierzu ist an der Innenseite der Folie, welche in der Zeichnung sichtbar geworden ist, eine aufkaschierte Schicht 14 vorhanden. Diese aufkaschierte Verstärkungsschicht besteht erfindungsgemäß aus Papier oder Vliesstoff, wobei vorzugsweise Papier oder Vliesstoffe der oben angegebenen Sorten und Flächengewichte verwendet werden. Wie erkennbar, befindet sich die Verstärkungsschicht 14 auf der Innenseite der WS-Folie 1, obgleich beim Gebrauch die Freienden 11 und 12 der Verschlußstreifen 7 und 8 auf die Außenseiten geklebt werden.

In der Zeichnung ist die Sache so dargestellt, daß die Verstärkungsschicht 14 ein durchgehender Streifen ist. Es ist dies nicht unerläßlich; die Verstärkungsschicht kann auch in zwei separate Etiketten unterteilt sein, die sich jeweils in denjenigen Flächenabschnitten befinden, an die die Freienden 11 und 12 der Verschlußstreifen erfahrungsgemäß geklebt werden.

**Patentansprüche**

1. Mehrmals verschließbare Windel mit
   - einer flüssigkeitsdurchlässigen oberen Abdeckung,
   - einer flüssigkeitsdichten unten angeordneten Wäscheschutzfolie (WS-Folie),
   - einer dazwischen angeordneten Saugschicht,
   sowie
   - wenigstens zwei Klebebändern an einem Ende der Windel, deren Freienden nach dem Anlegen der Windel an der äußeren Oberfläche der WS-Folie am gegenüberliegenden Ende der Windel ein- oder mehrmals festgeklebt werden,
   wobei
   - die WS-Folie dort, wo die Freienden der Klebebänder zum Verschließen der Windel festgeklebt werden, an der Folieninnenseite durch eine aufkaschierte Schicht verstärkt ist,
   **dadurch gekennzeichnet, daß**

   die auf der Innenseite der WS-Folie (1) angeordnete Verstärkungsschicht (14) aus Papier oder Vliesstoff besteht.

2. Windel nach Anspruch 1, **dadurch gekennzeichnet, daß** die Verstärkungsschicht (14) aus Papier mit einem Flächengewicht von 16 - 80 g/m$^2$ besteht.

3. Windel nach Anspruch 1 oder 2, **dadurch gekennzeichnet, daß** die Verstärkungsschicht (14) aus
   Seidenpapier mit 18 - 25 g/m$^2$ oder
   Kraftpapier mit 30 - 50 g/m$^2$ oder
   Pergaminpapier mit 25 - 40 g/m$^2$ oder
   satiniertem Papier mit 30 - 50 g/m$^2$ Flächengewicht besteht.

4. Windel nach Anspruch 1, **dadurch gekennzeichnet, daß** die Verstärkungsschicht (14) aus
   einem Zellwollvliesstoff mit 14 - 30 g/m$^2$ oder
   einem Polyestervliesstoff mit 10 - 30 g/m$^2$ oder
   einem Polyolefinvliesstoff mit 14 - 30 g/m$^2$ Flächengewicht besteht.

4

Europäisches
Patentamt

**EUROPÄISCHER RECHERCHENBERICHT**

Nummer der Anmeldung

EP    92 10 9432

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,X | EP-A-0 287 767 (KOESTER KG)<br>* das ganze Dokument *<br>--- | 1 | A61F13/58 |
| X | US-A-4 753 649 (P. A. PAZDERNIK)<br>* Spalte 3, Zeile 29 - Zeile 49 *<br>* Spalte 3, Zeile 59 - Spalte 4, Zeile 19 *<br>* Spalte 7, Zeile 63 - Spalte 8, Zeile 4 *<br>* Spalte 9, Zeile 42 - Zeile 63; Ansprüche 1-13; Abbildungen 1-5 *<br>--- | 1 | |
| A | EP-A-0 080 647 (BOUSSAC SAINT FRERES B.S.F.)<br>* Seite 8, Zeile 1 - Zeile 13; Ansprüche 1-4; Abbildungen 1-4 *<br>--- | 1 | |
| D,A | DE-A-3 022 916 (KIMBERLY-CLARK CORPERATION)<br>* Seite 10, Zeile 17 - Seite 11, Zeile 9; Ansprüche 1,2; Abbildungen 1-3 *<br>--- | 1 | |
| A | EP-A-0 259 075 (MINNESOTA MINING AND MANUFACTURING COMPANY)<br><br>----- | | |

**RECHERCHIERTE SACHGEBIETE (Int. Cl.5)**

A61F
A41B

Der vorliegende Recherchenbericht wurde für alle Patentansprüche erstellt

| Recherchenort | Abschlußdatum der Recherche | Prüfer |
|---|---|---|
| DEN HAAG | 14 SEPTEMBER 1992 | FAIRBANKS S.A. |